(19) Europäisches Patentamt
European Patent Office
Office européen des brevets

(11) Veröffentlichungsnummer: 0 326 890
A2

(12) EUROPÄISCHE PATENTANMELDUNG

(21) Anmeldenummer: 89101084.5

(51) Int. Cl.⁴: C08G 59/14 , C08G 59/50

(22) Anmeldetag: 23.01.89

Patentansprüche für folgenden Vertragsstaat: ES.

(30) Priorität: 04.02.88 DE 3803213

(43) Veröffentlichungstag der Anmeldung: 09.08.89 Patentblatt 89/32

(84) Benannte Vertragsstaaten: BE DE ES FR GB IT NL

(71) Anmelder: Th. Goldschmidt AG
Goldschmidtstrasse 100 Postfach 101461
D-4300 Essen 1(DE)

(72) Erfinder: Fock, Jürgen, Dr.
Mörsenbroicher Weg 114
D-4000 Düsseldorf 30(DE)
Erfinder: Esselborn, Eberhard
Pilotystrasse 21
D-4300 Essen 1(DE)
Erfinder: Nickel, Friedheim
Osterholzer Dorfstrasse 85
D-2800 Bremen 44(DE)

(54) **Polyoxyalkylenether mit quaternären Ammoniumgruppen.**

(57) Polyoxyalkylenether mit quaternären Ammoniumgruppen der allgemeinen durchschnittlichen Formel

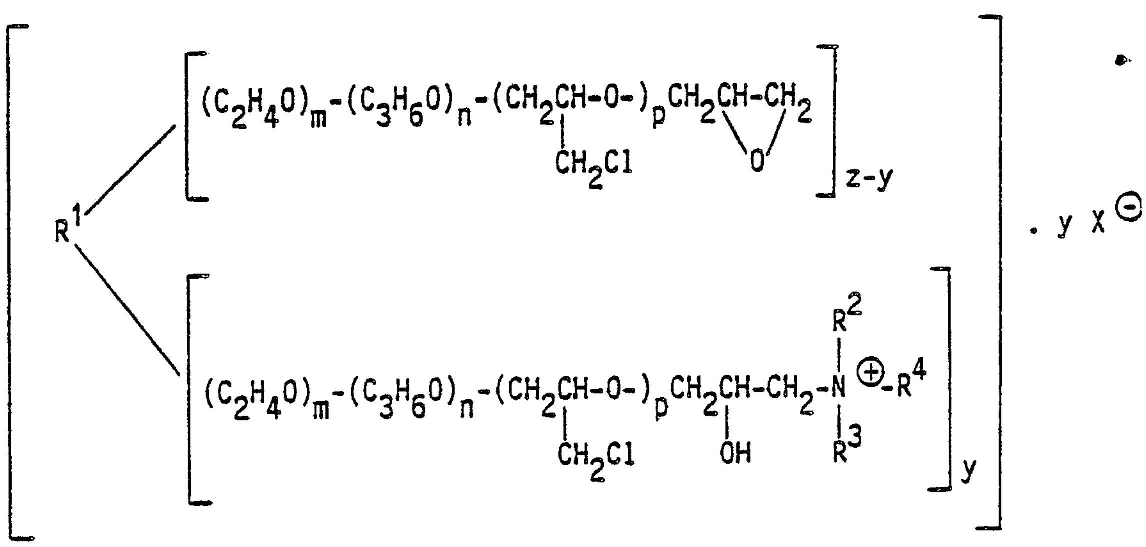

in der
$R^1$ ein z-wertiger aliphatischer Alkoxyrest,
z = 2 bis 9,
y = 1 bis 8,
z-y ≥ 1,
m = 2 bis 75,
n = 0 bis 10,

EP 0 326 890 A2

p = 0 bis 3,
$R^2$ ein Wasserstoffatom oder ein Alkyl- oder Phenylrest ist,
$R^3$, $R^4$ gleich oder verschieden sind und einen Alkyl-, Hydroxyalkyl-, Amidoalkyl- oder Phenylrest bedeuten oder zusammen mit dem Stickstoffatom gemeinsamer Bestandteil eines 5- oder 6-atomigen Ringsystems oder eines Chinolinringes sind,
X ein Halogen-Anion ist,
wobei die Alkylreste bis zu 18 Kohlenstoffatome aufweisen.

Die Erfindung betrifft ferner ein Verfahren zur Herstellung dieser Verbindung und Mittel zur permanenten antistatischen Ausrüstung von Fasern oder Faserprodukten in Form einer 0,1 bis 10 gew.-%igen wäßrigen Lösung eines Gemisches, welches die neuen Verbindungen und, bezogen auf Oxirangruppen, höchstens äquivalente Mengen eines Härters enthält.

## Polyoxyalkylenether mit quaternären Ammoniumgruppen

Die Erfindung betrifft Polyether mit quaternären Ammoniumgruppen, welche zusätzlich im Molekül eine oder mehrere Oxirangruppe(n) enthalten, ein Verfahren zur Herstellung dieser Verbindungen sowie ein Mittel zum permanenten antistatischen Ausrüsten von Fasern oder Faserprodukten, welche die neuen erfindungsgemäßen Verbindungen zusammen mit Härtern enthalten.

Zur antistatischen Ausrüstung von Fasern oder Faserprodukten werden in erheblichem Umfang Verbindungen mit quaternären Ammoniumgruppen verwendet. Diese Verbindungen sind im allgemeinen gut wasserlöslich oder wasserdispergierbar und weisen den Nachteil auf, daß sie keine permanenten Eigenschaften haben, d.h., daß sie von Waschflotten von der Faseroberfläche wieder abgelöst werden. Die vor der Wäsche antistatisch ausgerüsteten Fasern oder Faserprodukte verlieren deshalb nach ein oder mehreren Waschvorgängen ihre antistatischen Eigenschaften.

Der vorliegenden Erfindung liegt die Aufgabe zugrunde, Verbindungen aufzufinden, welche zur antistatischen Ausrüstung von Fasern oder Faserprodukten verwendet werden können, wobei diese Verbindungen aber derart auf der Faser fixiert werden, daß sie auch dem wiederholten Angriff von wäßrigen Waschflotten widerstehen. Gleichzeitig soll der Griff der ausgerüsteten Fasern oder Faserprodukte nicht negativ beeinflußt werden, wie dies bei vielen antistatisch wirkenden Mitteln des Standes der Technik zu beobachten ist. Es ist weiter erwünscht, daß die Fasern bei der Ausrüstung hydrophiliert werden, um die Trageeigenschaften der mit ihnen hergestellten Textilien zu verbessern.

Überraschenderweise wurde gefunden, daß dieses Eigenschaftsprofil, nämlich permanente antistatische Ausrüstung, guter Griff und Hydrophilierung der Faser, Polyoxyalkylenethern mit quaternären Ammoniumgruppen eigen ist, welche die allgemeine durchschnittliche Formel aufweisen:

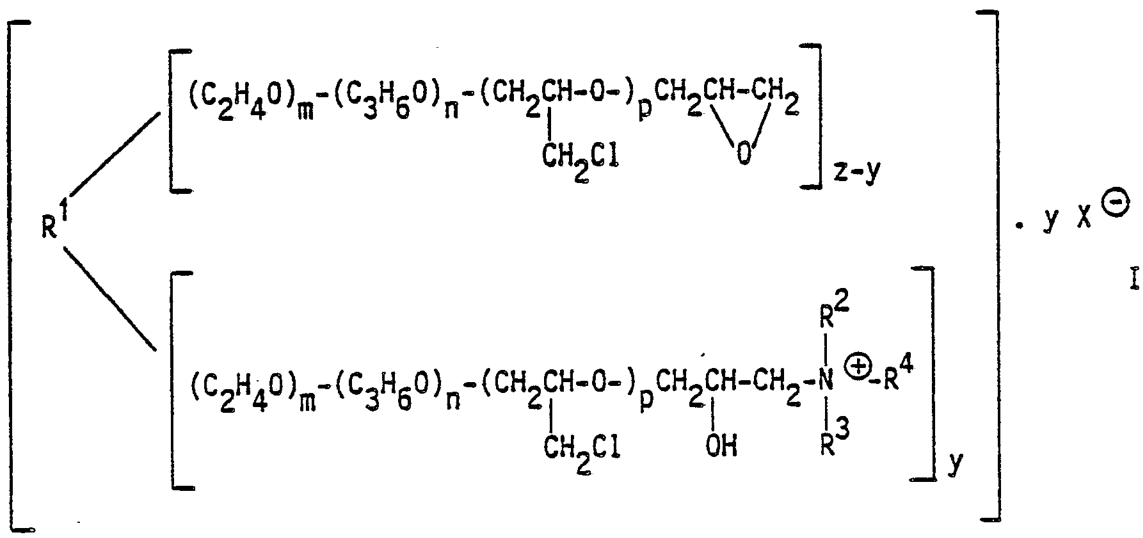

I

in der
$R^1$ ein z-wertiger aliphatischer Alkoxyrest,
z = 2 bis 9,
y = 1 bis 8,
z-y ≧ 1,
m = 2 bis 75,
n = 0 bis 10,
p = 0 bis 3,
$R^2$ ein Wasserstoffatom oder ein Alkyl- oder Phenylrest ist,
$R^3$, $R^4$ gleich oder verschieden sind und einen Alkyl-, Hydroxyalkyl-, Amidoalkyl- oder Phenylrest bedeuten oder zusammen mit dem Stickstoffatom gemeinsamer Bestandteil eines 5- oder 6-atomigen Ringsystems oder eines Chinolinringes sind,
X ein Halogen-Anion ist,
wobei die Alkylreste bis zu 18 Kohlenstoffatome aufweisen.

$R^1$ ist ein z-wertiger aliphatischer Alkoxyrest, wobei z einen Wert von 2 bis 9 haben kann. Beispiele für

solchen z-wertigen Alkoxyresten zugrunde liegenden Alkohole sind Ethylenglykol, Propylenglykol, Butandiol, Hexandiol, Glycerin, Trimethylolpropan, Pentaerythrit, Sorbit, Di-, Tri-, Tetra- oder Oligomere des Glycerins, des Glycidols, des Trimethylolpropans und des Pentaerythrits. An die OH-Gruppen dieser Alkohole sind Oxyethylen- und Oxypropyleneinheiten angelagert, wobei die Zahl der Oxyethyleneinheiten durch den Index m ausgedrückt ist und dieser Index m einen Wert von 2 bis 75 aufweist. Die Anzahl der Oxypropyleneinheiten wird durch den Index n wiedergegeben. n hat einen Wert von 0 bis 10. Die Oxypropyleneinheiten haben dabei insbesondere die Funktion, die Kristallisation der Polyether zu verhindern und sicherzustellen, daß das Copolymerisat in flüssiger Form vorliegt. Bei niedrigen Oxyethylengehalten kann deshalb auf einen Gehalt an Oxypropyleneinheiten verzichtet werden. p hat einen Wert von 0 bis 3.

Der Index y gibt die Anzahl der im durchschnittlichen Molekül enthaltenen Polyetherreste mit quaternären Ammoniumgruppen an. y hat einen Wert von 1 bis 8. Die Indices z und y sind durch die Bedingung miteinander verknüpft, daß z-y ≥ 1 ist. Hieraus ergibt sich, daß y einen Wert von 1 haben muß, wenn z 2 ist. Hat z einen Wert von 9, kann y einen Wert bis zu 8 annehmen.

Der Rest $R^2$ hat die Bedeutung eines Wasserstoffatoms oder eines Alkyl- oder Phenylrestes. Vorzugsweise ist er ein Wasserstoff- oder Alkylrest. Die Alkylreste können geradkettig oder verzweigt sein und bis zu 18 Kohlenstoffatome aufweisen. Vorzugsweise weisen sie 1 bis 12, insbesondere 1 bis 4 Kohlenstoffatome auf. Die Alkylreste können endständig eine Hydroxylgruppe tragen.

Die Reste $R^3$ und $R^4$, die gleich oder verschieden sind, können Alkyl-, Hydroxyalkyl-, Amidoalkyl- oder Phenylreste der bei $R^2$ beschriebenen Art sein. Die Reste $R^3$ und $R^4$ können außerdem zusammen mit dem Stickstoffatom gemeinsam Bestandteil eines 5- oder 6-atomigen Ringsystems oder des Chinolinringes sein und z.B. einen Imidazolinium-, Imidazolium- oder Pyridiniumring bilden. Beispiele entsprechend substituierter quaternärer Ammoniumgruppen sind

$-N^{\oplus}(CH_3)_3$, 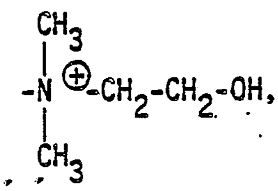 , $-N^{\oplus}(C_4H_9)_3$, 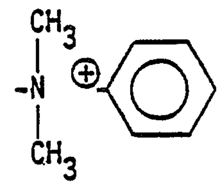 ,

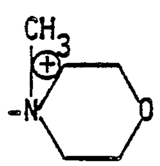 , 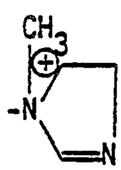 , 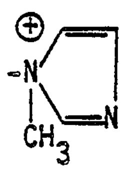 , 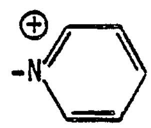 , 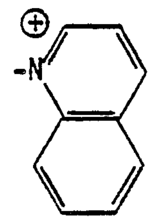 ,

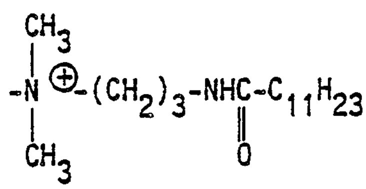 .

X ist ein Halogen-Anion, vorzugsweise ein Chloridion. Die Art dieses Anions ist von geringerer Bedeutung und oft herstellungsbedingt.

Die erfindungsgemäßen Verbindungen sind wasserlösliche bis wasserdispergierbare, mäßig viskose, farblose bis schwach gelblich gefärbte Flüssigkeiten.

Ein weiterer Gegenstand der Erfindung ist ein Verfahren zur Herstellung dieser Verbindungen mit dem Kennzeichen, daß man Verbindungen der allgemeinen durchschnittlichen Formel

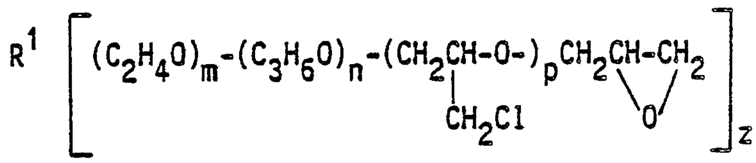 II

wobei $R^1$, m, n, p und z die bereits angegebene Bedeutung haben, mit y Mol des Hydrohalogenids eines tertiären Amins der Formel

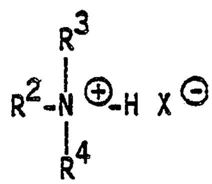

bei gegebenenfalls erhöhter Temperatur in an sich bekannter Weise umsetzt. (Methoden der Organischen Chemie, Houben-Weyl, Band 11/2, S. 610)

Die Herstellung der Ausgangsverbindungen II erfolgt in an sich bekannter Weise durch Anlagern von Ethylenoxid und gegebenenfalls Propylenoxid an den Ausgangsalkohol $R^1H$, Umsetzen des Anlagerungsproduktes mit Epichlorhydrin und anschließende Bildung der Oxirangruppe durch Umsetzung mit Natriummethylat.

Die erfindungsgemäße Umsetzung dieser Ausgangsverbindungen erfolgt mit y Mol des Hydrohalogenids eines tertiären Amins der Formel

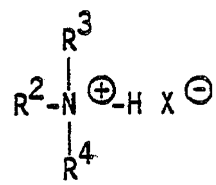

Die Umsetzung mit dem Salz des tertiären Amins läuft bereits bei Raumtemperatur ab. Sie kann gegebenenfalls durch Anwendung erhöhter Temperaturen weiter beschleunigt und vervollständigt werden. Eine Einführung von quaternären Ammoniumgruppen gelingt aber auch, wenn die nach der Umsetzung mit Epihalogenhydrin entstandene Halogenhydrinendgruppe direkt mit einem tertiären Amin zur Reaktion gebracht wird.

Entsprechend der der Erfindung zugrunde liegenden Aufgabenstellung ist ein weiterer Gegenstand vorliegender Erfindung die Bereitstellung eines Mittels zur permanenten antistatischen Ausrüstung von Fasern oder Faserprodukten in Form einer 0,1 bis 10 gew.-%igen wäßrigen Lösung eines Gemisches, welches Verbindungen nach Patentanspruch 1 und, bezogen auf Oxirangruppen, höchstens äquivalente Mengen eines an sich bekannten Härters enthält.

Als Härter eignen sich die aus dem Stand der Technik bekannten, mit Oxirangruppen reaktiven Vernetzungsmittel. Besonders geeignet sind dabei mehrwertige Amine, mehrwertige Amide, mehrwertige Thioalkohole oder anorganische oder organische Säuren. Beispiele solcher Härter sind Polyamine, wie Ethylendiamin, Diethylentriamin, Triethylentetramin und deren Umsetzungsprodukte mit Diglycidylethern von Bisphenol A, Polyalkylenoxid mit endständigen Aminogruppen, Dicyandiamid, Mercaptogruppen enthaltende Verbindungen, Polyaminoimidazoline, Perfluoralkylcarbonsäure, Perfluoralkylsulfonsäure, Phosphorsäure.

Besonders bevorzugte Vernetzer sind Amine der allgemeinen Formel

$R^5[(C_2H_4O)_aNH_2]_b$ oder $R^5[(C_3H_6O)_aNH_2]_b$

In dieser Formel ist $R^5$ ein b-wertiger Alkoholrest, wobei b 2, 3 oder 4 bedeutet. Beispiele solcher Alkoholreste sind Ethylenglykol, Propylenglykol, Butandiol, Glycerin oder Pentaerythrit, wobei jeweils das Wasserstoffatom der Hydroxylgruppen abgespalten ist. a ist eine Zahl von 4 bis 40.

Der Verarbeitungszeitraum der erfindungsgemäßen Mittel kann wesentlich verlängert werden, wenn man statt der freien Amine deren Ammoniumverbindungen, vorzugsweise deren Acetate, verwendet.

Die erfindungsgemäßen Mittel können zusätzliche Komponenten z.B. zur weiteren Beeinflussung des

Griffs, zur Erzielung schmutz- oder fettabweisender Eigenschaften oder zum Erhalt anderer Gebrauchseigenschaften enthalten.

Zur Ausrüstung der Fasern oder Faserprodukte kann man sich der üblichen Techniken, wie z.B. Tauchen, Besprühen, bedienen. Die Behandlung der Fasern erfolgt vorzugsweise in wäßrigen Lösungen, welche 0,1 bis 10 Gew.-% Verbindungen des Patentanspruchs 1 enthalten. Erwünscht ist dabei eine Aufnahme an Ausrüstungsmittel von 0,1 bis 5 %. Die behandelten Fasern werden beim oder nach dem Trocknen vorzugsweise auf Temperaturen von 100 bis 170 °C erwärmt. Dabei härten die auf der Faser befindlichen Polymerisate aus.

In den folgenden Beispielen wird die Herstellung der erfindungsgemäßen quaternäre Ammoniumgruppen aufweisenden Polyether gezeigt. Es wird ferner die Herstellung der erfindungsgemäßen Mittel und deren Verwendung zur Ausrüstung von Textilgut näher erläutert.

A) Herstellung eines oxiranylfunktionellen Polyethers

182 g (ca. 1 Mol) fein gepulvertes Sorbit, das in 150 g Verfahrensprodukt angeteigt worden ist, und 11,2 g (ca. 0,2 Mol) Kaliumhydroxid werden sorgfältig vermischt in einen Reaktor gegeben. Nach ausgiebiger Spülung mit Reinstickstoff wird auf 110 °C erhitzt und dazu werden 2379 g (ca. 54 Mol) Ethylenoxid so schnell zugegeben, daß die Reaktorinnentemperatur 120 °C und der Druck 6 bar nicht überschreiten. Nach vollständiger Einleitung des Ethylenoxids wird die Temperatur so lange auf 115 °C gehalten, bis ein gleichbleibender Druck das Ende der Nachreaktion anzeigt. Danach werden bei 80 bis 90 °C die Restmonomere durch Evakuieren entfernt.

Das erhaltene Produkt wird mit Hilfe von verdünnter Phosphorsäure neutralisiert und das Wasser durch Destillation, das entstandene Natriumphosphat durch Filtration zusammen mit einem Filterhilfsmittel entfernt. Die Hydroxylzahl des Verfahrensproduktes beträgt 171, was bei einer angenommenen Funktionalität von 6 einem Molekulargewicht von 1970 entspricht.

1970 g (ca. 1 Mol) des erhaltenen Polyethers werden zusammen mit 5,6 g Zinn-IV-chlorid auf 80 °C erwärmt. Dazu werden 832,5 g (ca. 9 Mol) Epichlorhydrin über einen Zeitraum von 1/2 h unter Einhaltung der Temperatur getropft. Anschließend wird noch zweimal im Abstand von 30 min mit je 5,6 g $SnCl_4$ nachkatalysiert sowie für 2 h Gelegenheit zur Nachreaktion gegeben. In einer zweiten Reaktionsstufe werden tropfenweise 340,2 g (ca. 6,3 Mol) Natriummethylat, gelöst in 793 g Methanol, zugegeben und das Gemisch für 2 h bei 25 °C gehalten.

Nach Entfernung der flüchtigen Bestandteile durch Destillation wird das angefallene Natriumchlorid durch Filtration entfernt. Die Epoxidzahl des erhaltenen Produktes wird zu 3,64 in Gew.-% aktiver Sauerstoff errechnet (Polyether 1).

In analoger Weise werden die Polyether 2 bis 7 hergestellt. Deren Zusammensetzung ergibt sich aus Tabelle 1.

Tabelle 1

| Polyether Nr. | Starteralkohol | Ethylenoxid [Mol] | Propylenoxid [Mol] | Molekulargewicht OHZ* | Epoxidzahl Gew.-% 0 |
|---|---|---|---|---|---|
| 1 | Sorbit | 45,0 | - | 1970 | 3,64 |
| 2 | Butandiol-1,4 | 36,9 | 4,9 | 1920 | 1,50 |
| 3 | Pentaerythrit | 21,0 | - | 1080 | 4,13 |
| 4 | Pentaerythrit | 71,0 | 8,0 | 3180 | 1,61 |
| 5 | Sorbit | 44,8 | 6,0 | 2240 | 3,09 |
| 6 | Sorbit | 22,0 | - | 1100 | 5,72 |
| 7 | Polyglycerin Molekulargewicht ca. 500 | 82,0 | 11,0 | 4280 | 2,43 |

* unmittelbar nach der Alkoxylierung

B) Herstellung eines quaternäre Ammoniumgruppen enthaltenden oxiranylfunktionellen Polyethers

440 g(ca. 1 Mol) des gemäß A) erhaltenen oxiranylierten Polyethers werden in einem Reaktor unter

Stickstoff auf 80 °C erhitzt und dazu ein Addukt aus 44,5 g (ca. 0,5 Mol) N,N-Dimethylaminoethanol und 50,7 g 36 %iger Salzsäure gegeben. Die Umsetzung zur quaternären Ammoniumgruppe ist nach etwa 2 h beendet, was sich an der dann gleichbleibenden Epoxidzahl erkennen läßt. Der Epoxidgehalt des erhaltenen Produktes beträgt 1,56 %, was bei einer angenommenen Funktionalität von 3 einer Ausbeute von 97,8 % der Theorie entspricht.

In analoger Weise werden andere oxiranylfunktionelle Polyether aus der Tabelle 1 mit verschiedenen tertiären Aminen umgesetzt. In der Tabelle 2 werden die resultierenden Addukte und deren analytische Daten wiedergegeben.

Tabelle 2

| Addukt Nr. | Polyether Nr. | Aminhydrochlorid | Aminhydrochlorid/Polyether [Mol / Mol] | Epoxidzahl Gew.-% O |
|---|---|---|---|---|
| 1 | 1 | DMEA | 3 / 1 | 1,62 |
| 2 | 1 | DMTDA | 3 / 1 | 1,40 |
| 3 | 1 | TEA | 3 / 1 | 1,55 |
| 4 | 1 | TMA | 3 / 1 | 1,66 |
| 5 | 1 | LAPDMA | 3 / 1 | 1,45 |
| 6 | 2 | DMEA | 1 / 1 | 0,72 |
| 7 | 3 | DMEA | 2 / 1 | 1,86 |
| 8 | 4 | DMEA | 2 / 1 | 0,76 |
| 9 | 5 | DMEA | 3 / 1 | 1,36 |
| 10 | 6 | MORPH | 3 / 1 | 2,25 |
| 11 | 6 | PYR | 3 / 1 | 2,45 |
| 12 | 6 | CHIN | 3 / 1 | 2,15 |
| 13 | 6 | MIMI | 3 / 1 | 2,30 |
| 14 | 6 | DMEA | 5 / 1 | 0,72 |
| 15 | 6 | DMEA | 4 / 1 | 1,53 |
| 16 | 6 | DMEA | 3 / 1 | 2,30 |
| 17 | 6 | DMEA | 2 / 1 | 3,25 |
| 18 | 6 | DMEA | 1 / 1 | 4,50 |
| 19 | 6 | - | 0 / 1 | 5,72 |
| 20 | 7 | DMEA | 5 / 1 | 0,88 |
| 21 | 1 | MDEA | 3 / 1 | 1,58 |

| | | |
|---|---|---|
| DMEA | = | Dimethylaminoethanol |
| MDEA | = | Methyldiethanolamin |
| DMTDA | = | Dimethyltetradecylamin |
| TEA | = | Triethanolamin |
| TMA | = | Trimethylamin |
| LAPDMA | = | N,N,N-Dimethyllauroylamidopropylamin |
| PYR | = | Pyridin |
| CHIN | = | Chinolin |
| MORPH | = | N-Methylmorpholin |
| MIMI | = | N-Methylimidazolin |

Herstellung der erfindungsgemäßen Mittel und anwendungstechnische Ausprüfung

Die erfindungsgemäßen Mittel werden durch gemeinsames Lösen des quaternäre Ammoniumgruppen enthaltenden Adduktes und eines Vernetzers in Wasser hergestellt. Im Fall der vernetzenden Komponente handelt es sich im konkreten Fall entweder um ein $\alpha,\omega$-Diaminopolyethylenoxid mit einem Molekulargewicht von ca. 600 (Nr. 1) oder um Triethylentetramin (Nr. 2). Die wäßrige Lösung hat eine Konzentration von etwa 2 %. Die textiltechnische Prüfung erfolgt mit Hilfe von Geweben aus Polyester, die nach Tränkung mit der wäßrige Lösung und Abquetschen zwischen zwei Gummiwalzen bei 80° C getrocknet und anschließend für 10 min bei 140° C mit dem Ziel der Vernetzung erhitzt werden. Die Ermittlung des Oberflächenwiderstandes der mit den erfindungsgemäßen Mitteln antistatisch ausgerüsteten Gewebeprüflinge erfolgt gemäß DIN 53482 / VDE 0303 Teil 3 unter Verwendung einer Ringelektrode bei einer Temperatur von 20° C und einer relativen Luftfeuchtigkeit von 50 %. Die Zusammensetzung der erfindungsgemäßen Mittel, die Gewebeauflage und der Oberflächenwiderstand vor und nach der Extraktion werden in Tabelle 3 beschrieben.

Tabelle 3

| Mittel Nr. | Addukt Nr. | Vernetzer*) | Auflage bez. auf Gewebe [%] | Oberflächenwiderstand | |
|---|---|---|---|---|---|
| | | | | vor Extraktion | nach Extraktion |
| | | | | [Ω] • $10^{-7}$ | [Ω] • $10^{-7}$ |
| 1 | 1 | 1 | 3,1 | 20 | 190 |
| 2 | 2 | 1 | 2,1 | 15 | 190 |
| 3 | 3 | 1 | 2,2 | 20 | 4000 |
| 4 | 4 | 2 | 2,6 | 60 | 130 |
| 5 | 5 | 2 | 2,8 | 5 | 130 |
| 6 | 6 | 2 | 2,5 | 30 | 1500 |
| 7 | 7 | 2 | 2,2 | 20 | 300 |
| 8 | 8 | 2 | 2,0 | 25 | 900 |
| 9 | 9 | 1 | 2,8 | 20 | 150 |
| 10 | 10 | 2 | 1,9 | 50 | 250 |
| 11 | 11 | 2 | 2,1 | 100 | 2300 |
| 12 | 12 | 2 | 2,2 | 500 | 1200 |
| 13 | 13 | 2 | 2,4 | 15 | 200 |
| 14 | 14 | 2 | 2,0 | 5 | 150 |
| 15 | 15 | 2 | 1,9 | 15 | 90 |
| 16 | 16 | 2 | 2,0 | 50 | 200 |
| 17 | 17 | 2 | 2,0 | 100 | 350 |
| 18 | 18 | 2 | 2,1 | 120 | 200 |
| 19 | 19 | 2 | 2,0 | 500 | 500 |
| 20 | 20 | 2 | 2,0 | 25 | 95 |
| 21 | 1 | 2 | 2,4 | 25 | 40 |
| 22 | 21 | 2 | 2,2 | 25 | 40 |

*) α,ω-Diaminopolyethylenoxid (Molekulargewicht ≈ 600) = 1 Tripropylentramin = 2

## Ansprüche

1. Polyoxyalkylenether mit quaternären Ammoniumgruppen der allgemeinen durchschnittlichen Formel

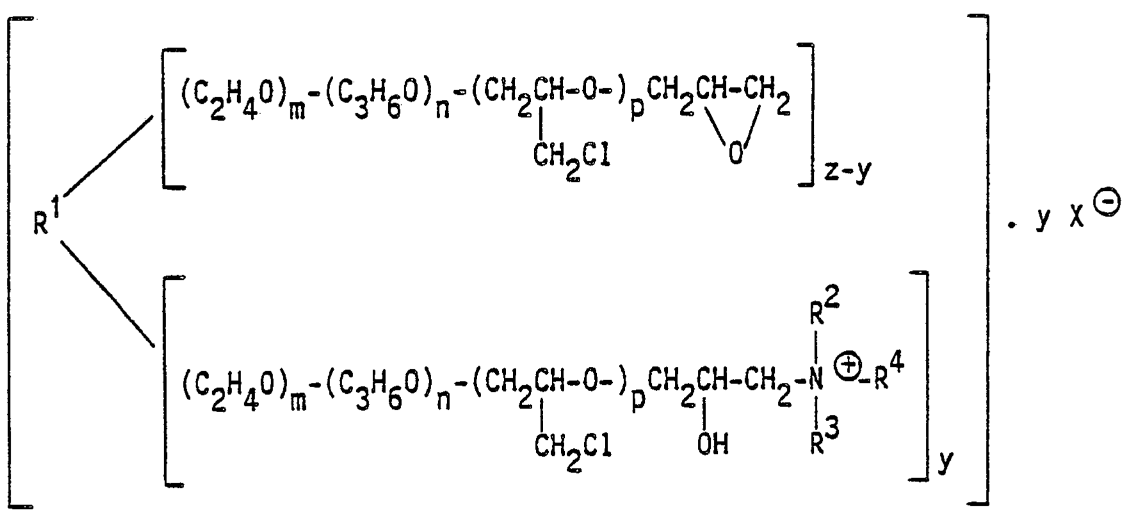

in der
$R^1$ ein z-wertiger aliphatischer Alkoxyrest,
z = 2 bis 9,
y = 1 bis 8,
z-y ≥ 1,
m = 2 bis 75,
n = 0 bis 10,
p = 0 bis 3,
$R^2$ ein Wasserstoffatom oder ein Alkyl- oder Phenylrest ist,
$R^3$, $R^4$ gleich oder verschieden sind und einen Alkyl-, Hydroxyalkyl-, Amidoalkyl- oder Phenylrest bedeuten oder zusammen mit dem Stickstoffatom gemeinsamer Bestandteil eines 5- oder 6-atomigen Ringsystems oder eines Chinolinringes sind,
X ein Halogen-Anion ist,
wobei die Alkylreste bis zu 18 Kohlenstoffatome aufweisen.

2. Verfahren zur Herstellung der Verbindungen nach Anspruch 1, dadurch gekennzeichnet, daß man Verbindungen der allgemeinen durchschnittlichen Formel

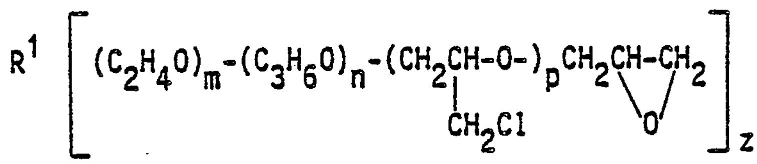

wobei $R^1$, m, n, p und z die bereits angegebene Bedeutung haben, mit y Mol des Hydrohalogenids eines tertiären Amins der Formel

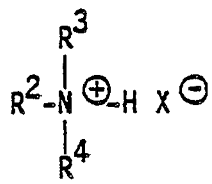

bei gegebenenfalls erhöhter Temperature in an sich bekannter Weise umsetzt.

3. Mittel zur permanenten antistatischen Ausrüstung von Fasern oder Faserprodukten in Form einer 0,1 bis 10 gew.-%igen wäßrigen Lösung eines Gemisches, welches Verbindungen nach Patentanspruch 1 und, bezogen auf Oxirangruppen, höchstens äquivalente Mengen eines an sich bekannten Härters enthält.

Patentansprüche für folgenden Vertragsstaat: ES

1. Verfahren zur Herstellung von Polyoxyalkylenethern mit quaternären Ammoniumgruppen der allgemeinen durchschnittlichen Formel

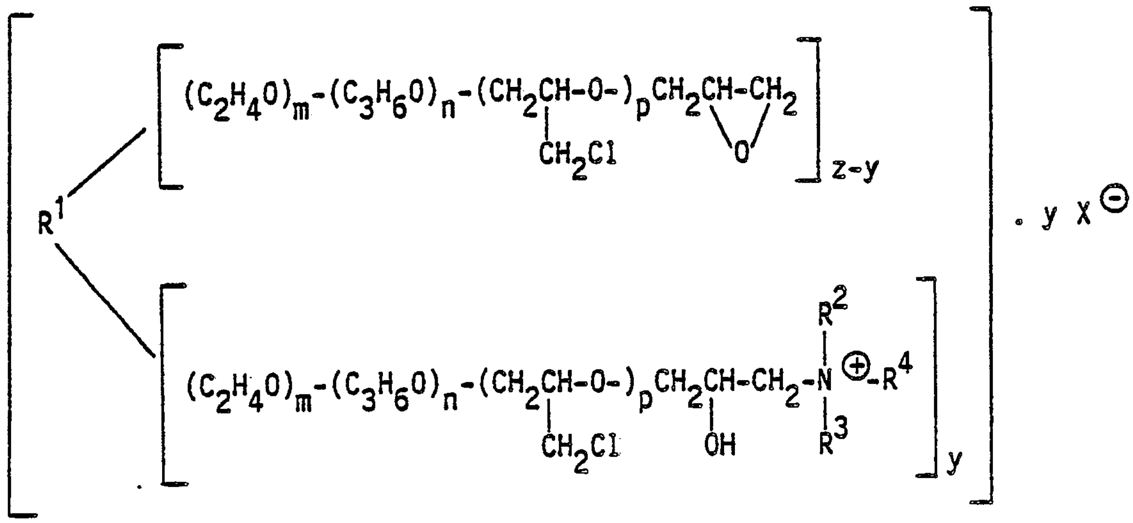

in der

$R^1$ ein z-wertiger aliphatischer Alkoxyrest,

z = 2 bis 9,

y = 1 bis 8,

z-y ≧ 1,

m = 2 bis 75,

n = 0 bis 10,

p = 0 bis 3,

$R^2$ ein Wasserstoffatom oder ein Alkyl- oder Phenylrest ist,

$R^3$, $R^4$ gleich oder verschieden sind und einen Alkyl-, Hydroxyalkyl-, Amidoalkyl- oder Phenylrest bedeuten oder zusammen mit dem Stickstoffatom gemeinsamer Bestandteil eines 5- oder 6-atomigen Ringsystems oder eines Chinolinringes sind,

X ein Halogen-Anion ist,

wobei die Alkylreste bis zu 18 Kohlenstoffatome aufweisen,

dadurch gekennzeichnet, daß man Verbindungen der allgemeinen durchschnittlichen Formel

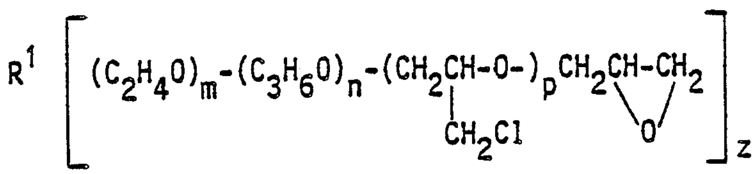

wobei $R^1$, m, n, p und z die bereits angegebene Bedeutung haben, mit y Mol des Hydrohalogenids eines tertiären Amins der Formel

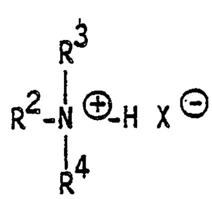

bei gegebenenfalls erhöhter Temperatur in an sich bekannter Weise umsetzt.

2. Mittel zur permanenten antistatischen Ausrüstung von Fasern oder Faserprodukten in Form einer 0,1 bis 10 gew.-%igen wäßrigen Lösung eines Gemisches, welches Verbindungen, die nach Patentanspruch 1 hergestellt sind, und, bezogen auf Oxirangruppen, höchstens äquivalente Mengen eines an sich bekannten Härters enthält.